# EUROPEAN PATENT SPECIFICATION

(11) **EP 3 315 945 B1**
(45) Date of publication and mention of the grant of the patent: **08.07.2020**
(21) Application number: 16813639.8
(22) Date of filing: 27.05.2016
(51) Int. Cl.: G01N 21/78, G01N 33/84, G01N 21/33

(54) **METHOD FOR MEASURING BISMUTH CONTENT IN COLLOIDAL BISMUTH PECTIN OR COLLOIDAL BISMUTH PECTIN-CONTAINING PREPARATION**
VERFAHREN ZUR MESSUNG VON BISMUTGEHALT IN KOLLOIDALEM BISMUTPEKTIN ODER IN KOLLOIDALEM BISMUTPEKTINHALTIGEM PRÄPARAT
PROCÉDÉ DE MESURE DE LA TENEUR EN BISMUTH DANS DE LA PECTINE DE BISMUTH COLLOÏDAL OU DANS UNE PRÉPARATION CONTENANT DE LA PECTINE DE BISMUTH COLLOÏDAL

(30) Priority: 23.06.2015 CN 201510348311
(43) Date of publication of application: 02.05.2018
(73) Proprietor: Shanxi Zhendong Ante Biopharmaceutical Co. Ltd., Jinzhong, Shanxi 030600 (CN)
(72) Inventor: LI, Anping, Jinzhong Shanxi 030600 (CN); CUI, Feng, Jinzhong Shanxi 030600 (CN); ZHU, Ping, Jinzhong Shanxi 030600 (CN); QIN, Zhengguo, Jinzhong Shanxi 030600 (CN); ZHENG, Tai, Jinzhong Shanxi 030600 (CN); WU, Yuexia, Jinzhong Shanxi 030600 (CN)
(74) Representative: Klunker IP Patentanwälte PartG mbB
(86) International application number: PCT/CN2016/083739
(87) International publication number: WO 2016/206523

(56) References cited:
- CN-A- 1 088 437
- CN-A- 102 507 381
- CN-A- 102 590 123
- CN-A- 104 880 428
- CN-A- 104 897 668
- SU-A1- 1 205 012
- George Norwitz ET AL: "SPECTROPHOTOMETRIC DETERMINATION OF BISMUTH IN COPPER AND CARTRIDGE BRASS BY THE IODIDE METHOD", Analytica Chimica Acta, Elsevier Scientific Publishing Company, 1976, pages 289-295, XP055514954, Amsterdam, Netherlands Retrieved from the Internet: URL:https://ac.els-cdn.com/S00032670018465 59/1-s2.0-S0003267001846559-main.pdf?_tid= ec34955c-bc0f-47ed-973a-cb56421b6ba2&acdna t=1539347824_3743db0e2bd1f92193d4dbf38abb5 2a5
- SONG YA-RU; WANG SHANG-ZHI; WANG DE-FA; NIU LI-PING: "Determination of bismuth pectin by flow-injection hydride-generation atomic absorption spectrometry", GUANG PU XUE YU GUANG PU FEN XI = GUANG PU CHINA DEC 2002, vol. 22, no. 6, December 2002 (2002-12), pages 1043-1044, XP009508636, China ISSN: 1000-0593
- GE, XIAOMING et al.: "Determination of Bismuth in Compound Tetracycline Hydrochloride Capsules by Spectrophotometry", CHINESE JOURNAL OF PHARMACEUTICAL ANALYSIS, vol. 25, no. 6, 30 June 2005 (2005-06-30), pages 670-672, XP009507902,
- Methods for Chemical Analysis of Tin-Determination of Bismuth Content, 17 December 2013 (2013-12-17), page 1, XP009507862,
- Anonymous: "Methods for Chemical Analysis of Tin-Determination of Bismuth Content", Chinese Standard, 17 December 2013 (2013-12-17), pages 1-12, XP009507862,
- MADRAKIAN, T. et al.: "Spectrophotometrie Determination of Bismuth in Water Samples after Preconcentration of its Thiourea-Bromide Ternary Complex on Activated Carbon", TALANTA, vol. 60, 31 December 2003 (2003-12-31), pages 831-838, XP055362807,
- WU, TAIBAI;: "Dual-Wavelength Spectrophotometric Determination of Bi(III) with CPB Triton X-100-BPR", METALLURGICAL ANALYSIS, vol. 8, no. 1, 31 January 1988 (1988-01-31), pages 15-18, XP055504732,
- GUMUS, G. et al.: "Determination of Bismuth and Zinc in Pharmaceuticals by First Derivative UV-Visible Spectrophotometry", ANALYTICA CHIMICA ACTA, vol. 547, 8 April 2005 (2005-04-08), pages 138-143, XP027730945,

## Description

### BACKGROUND OF THE PRESENT INVENTION

### Field of Invention

The present invention relates to a method for measuring a bismuth content, and more particularly to a method for measuring a bismuth content in polymeric chelating bismuth-contained substances.

### Description of Related Arts

The colloidal bismuth pectin is a compound of uncertain constitution of the pectin and the bismuth Bi, which is yellow powder; wherein the bismuth pectin content (takes bismuth for calculation) is 14.0%-16.0%; pH is 8.5-10.5; the sedimentation rate is 1-0.97. The colloidal bismuth pectin is insoluble in ethanol, acetone, ether and other organic solvents, which is able to disperse uniformly and form stable colloidal system in the water.

The colloidal bismuth pectin substitutes small molecule acid groups with biological macromolecules pectin. Compared with other bismuth preparations such as bismuth subgallate, bismuth subnitrate, bismuth subsalicylate and bismuth potassium cirtrate, the colloidal bismuth pectin has strong colloidal characters, high viscosity and low absorption by human body. The colloidal pectin bismuth has high affinity for ulcerated mucosa, which forms chelation with the ulcer mucoprotein by bismuth to cover on the gastric mucosa. The epithelial tissues are stimulated to discharge mucus and the pepsin activity is inhibited to protect the gastric mucosa. The bismuth is able to kill the *Helicobacter pylori.* Compared to the conventional medicine, the colloidal bismuth pectin has strong protection for mucosa, which has been widely applied in the treatment of intestinal tract disease including peptic ulcer disease, chronic gastritis and etc.

The colloidal bismuth pectin and the capsule preparation is an original ground medicine manufactured by the Taiyuan Red Star Pharmacy Plant which is the predecessor of Shanxi Zhendong Ante Biopharmaceutical Co., Ltd. The medicine gets the new drug certification and the drug production license from the Ministry of Health of the People's Republic of China in 1992 and now is listed in the Pharmacopoeia of the People's Republic of China, 2nd volume of the 2010 version. The method for measuring the colloidal bismuth pectin content is same with the method for the bismuth subnitrate, bismuth subsalicylate, bismuth potassium cirtrate and etc., which is to dissolve the test article with nitric acid by heating, indicate with xylenol orange indicator, adopt complexometric titration, titrate the solution with the EDTA-2Na (ethylenediaminetetraacetic acid disodium salt dehydrate) volumetric solution until the solution shows yellow. Due to the biomacromolecule character of the colloidal bismuth pectin, an opaque turbid colloidal system tends to form in the measuring process; the xylenol orange is used for both the complexometric indicator and acid-base indicator; the color change of the indicator tends to be disturbed and is hard to be keenly observed, which effects the end point detection and compromises the repeatability and accuracy of the measurement method.

Ge xiaoming (Determination of Bismuth in Compound Tetracycline Hydrochloride Capsules by Spectrophotometry, Chinese Journal of Pharmaceutical Analysis, 2005, 25(6), 670-672) adopted ultraviolet-visible spectrophotometry to measure the bismuth salts content in the compound tetracycline hydrochloride capsule. The adopted reference article of bismuth potassium cirtrate is difficult to accurately valuate, which is not able to satisfy the accuracy requirement of the medicine content measurement method. The bismuth potassium cirtrate is a small molecule bismuth-contained compound and easy to dissolve in water, the acid group of which does not disturb the measurement. The colloidal bismuth pectin is polymeric bismuth-contained compound. The chelating between the pectin and bismuth is strong, which causes difficulty in bismuth dissolution. Measuring directly with the ultraviolet-visible spectrophotometry causes serious polymeric acid group pectin disturbance. So, the complexometric titration is adopted conventionally for the measurement of bismuth content in the colloidal bismuth pectin while the simple and accurate ultraviolet-visible spectrophotometry method is hard to use.

CN 102590 123 A discloses a method for detecting the content of bismuth in a bismuth potassium citrate preparation.

### SUMMARY OF THE PRESENT INVENTION

An object of the present invention is to provide a method based on the spectrophotometry for measuring the bismuth content in the colloidal bismuth pectin and the colloidal bismuth pectin-contained preparation, which is able to improve the repeatability and accuracy in measuring the bismuth content in the colloidal bismuth pectin and the colloidal bismuth pectin-contained preparation.

The present invention is defined in claim 1.

The protonic acid dissociation agent is nitric acid, hydrochloric acid or sulfuric acid. Optimally, the protonic acid dissociation agent is nitric acid.

The dispersion dissociated with the protonic acid is centrifuged for 5-15 minutes at a speed of 7000-10000r/min. The dissociated polymeric pectin in the dispersion is fully settled to form a bismuth test solution without disturbance which fulfils the test requirement of the spectrophotometry.

The chromogen solution is a water solution or a 0.2-2mol/L nitric acid solution of a potassium iodide, in which the citric acid or the ascorbic acid are added.

Furthermore, the chromogen solution comprises the citric acid or the ascorbic acid of 0.5wt%-10wt%, the potassium iodide of 2.5wt%-25wt%.

Optimally, the chromogen solution comprises the citric acid or the ascorbic acid of 2.5wt%, the potassium iodide of 12.5wt%.

The reference solution of the bismuth with a suitable concentration is prepared by dissolving the bismuth with the nitric acid before being diluted with water and adding the chromogen solution.

Specifically,in each 1ml test solution or the 1ml reference solution of the bismuth, the bismuth content is 0.1-50µg; optimally, the in each 1ml test solution or the 1ml reference solution of the bismuth, the bismuth content is 2-20µg; more optimally, in each 1ml test solution or the 1ml reference solution of the bismuth, the bismuth content is 5-12µg.

A single-wavelength method is adopted for measurement of the bismuth content. A double-wavelength method is also able to be adopted to avoid disturbance.

The yellow bismuth potassium iodide generated by the bismuth and the potassium iodide has characteristic absorption spectroscopy at 399±2nm(crest), 433±2nm(trough), 463±2nm(crest). When the single-wavelength method is adopted for measurement; detection wavelengths are arbitrarily chosen any one wavelength from 399nm, 433nm and 463nm, wherein optimally, 463nm is chosen.

When a double-wavelength method is adopted for measurement; detection wavelengths are arbitrarily chosen any two wavelengths from 399nm, 433nm and 463nm; wherein the content is calculated with absorbance difference; optimally, the combination of 433nm and 463nm is chosen.

The method for measurement of bismuth content is for colloidal bismuth pectin prepared by varied methods and any colloidal bismuth pectin-contained single or compound preparation, wherein the suitable preparations comprises tablets, dispersible tablets, granules, eneric-coated tablets, colon-enteric-coated tablets, capsules, eneric-coated capsules, colon-enteric-coated capsules and dry suspensions.

The benefits of the present invention are as follow.

The present invention aims at solving the problem of quality standard for the colloidal bismuth pectin and colloidal bismuth pectin-contained preparation and uses the colloidal bismuth pectin character of forming stable colloidal system in water. First forming stable colloidal solution by adding water; then adding protonic acid into the colloidal solution until an appropriate hydrogen ion concentration is formed for the dissociation of the colloidal bismuth pectin; completely dissociating the bismuth; separating the pectin sediment completely by centrifuging; avoiding the disturbance of the pectin for the measurement of absorbance, which realizes the bismuth content measurement by the ultraviolet-visible spectrophotometry method.

Furthermore, the present invention takes the advantage of the characteristic reaction between bismuth and potassium iodide in acid medium, which forms yellow bismuth potassium iodide. Based on the reaction, the bismuth content measurement by the ultraviolet-visible spectrophotometry method is established, which is able to accurately measure the bismuth content in the colloidal bismuth pectin and the colloidal bismuth pectin-contained preparation.

The present invention adopts the diluted solution of bismuth dissolving by nitric acid as the bismuth reference solution. The purity of the bismuth is over 99.99%, Compared to mineral salts such as the bismuth nitrate, the solution is more suitable to be the reference solution.

The present invention provides a method for measuring bismuth content in the colloidal bismuth pectin and the colloidal bismuth pectin-contained preparation. The method has strong specificity, high accuracy, good repeatability, good linear relationship and high sensitivity, which is able to act as a quality control method for bismuth in a colloidal bismuth pectin or a colloidal bismuth pectin-contained preparation to effectively control the product quality.

### DETAILED DESCRIPTION OF THE PREFERRED EMBODIMENT

Embodiment 1: colloidal bismuth pectin.
   1) Preparation of a chromogen solution: taking 5g ascorbic acid and 25g potassium iodide to place in a 200ml volumetric flask; adding 100ml of water; shaking to dissolve; adding 25ml of 1mol/L nitric acid solution; adding water to dilute; dripping water to meet a scale; thus, the chromogen solution containing 2.5% of ascorbic acid, 12.5% of potassium iodide is prepared.
   2) Preparation of a reference solution of bismuth: taking 275mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 2ml of standard bismuth stock solution to place in a 100ml volumetric flask; diluting with a nitric acid solution of 1mol/L until a scale is reached; thus, a solution containing about 55µg bismuth in each 1ml is prepared as a standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 37mg colloidal bismuth pectin powder to place in a 100ml volumetric flask; adding 50ml of water; shaking or impacting with an ultrasonic treatment to make the solution dissolve evenly; diluting with water until a scale is reached; thus, a colloidal solution containing about 55µg bismuth in each 1ml is prepared as a test stock solution; accurately weighing 5ml of the test stock solution to place in a 15ml centrifugal tube; accurately adding 5ml of 2mol/L nitric acid solution; shaking for 5 minutes; centrifuging for 10 minutes at 8000r/min; accurately measuring 5ml supernatant to place in a 25ml volumetric flask; diluting with chromogen solution until a scale is reached; thus, the test solution is prepared.
   4) Preparation of a blank solution: accurately measuring 5ml of water to place in a 15ml centrifugal tube; accurately adding 5ml of 2mol/L nitric acid solution; shaking for 5 minutes; accurately measuring 5ml of the solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying a ultraviolet-visible spectrophotometry, comprising steps of using 1cm quartz cuvette; measuring the absorbance at 463nm wavelength; calculating a colloidal bismuth pectin content with an external standard method; a result is 14.9% (take bismuth for calculation).
Embodiment 2: colloidal bismuth pectin capsule (specification: 40mg, take the bismuth for calculation).
   1) Ppreparation of a chromogen solution: taking 2.5g ascorbic acid and 12.5g potassium iodide to place in a volumetric flask of 200ml; adding 100ml of water; shaking to dissolve; adding 25ml of 1mol/L nitric acid solution; adding water to dilute; dripping water to meet the scale; thus, the chromogen solution containing 1.25% of the ascorbic acid, 6.25% of the potassium iodide is prepared.
   2) Preparation of a reference solution of bismuth: taking 250mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 1ml of the standard bismuth stock solution to place in a 10ml volumetric flask; diluting with a nitric acid solution of 1.2mol/L until a scale is reached; thus, a solution containing about 250µg bismuth in each 1ml is prepared as a standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 510mg content in a colloidal bismuth pectin capsule to place in a 100ml volumetric flask; adding 50ml of water; shaking or impacting with an ultrasonic treatment to make the solution dissolve evenly; diluting with water until a scale is reached; thus, a dispersion containing about 500µg bismuth in each 1ml is prepared as a test stock solution; accurately weighing 5ml of test stock solution to place in a 15ml centrifugal tube; accurately adding 5ml of 2.4mol/L nitric acid solution; shaking for 5 minutes; centrifuging for 15 minutes at 7000r/min; accurately measuring 5ml supernatant to place in a 25ml volumetric flask; diluting with the chromogen solution until a scale is reached; thus, the test solution is prepared.
   4) Preparation of a blank solution: accurately measuring 5ml of water to place in a 15ml centrifugal tube; accurately adding 5ml of 2.4mol/L nitric acid solution; shaking for 5 minutes; accurately measuring 5ml of a solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying a ultraviolet-visible spectrophotometry, comprising steps of using 1cm quartz cuvette; measuring an absorbance at 433nm wavelength; calculating a colloidal bismuth pectin content with an external standard method; a result is 100.2% of the labeled amount(take bismuth for calculation).
Embodiment 3: colloidal bismuth pectin capsule (specification: 50mg, take the bismuth for calculation).
   1) Preparation of a chromogen solution: taking 10g ascorbic acid and 50g potassium iodide to place in a volumetric flask of 200ml; adding 100ml of water; shaking to dissolve; adding 25ml of 1mol/L nitric acid solution; adding water to dilute; dripping water to meet the scale; thus, the chromogen solution containing 5% of an ascorbic acid, 25% of a potassium iodide is prepared.
   2) Preparation of a reference solution of bismuth: taking 500mg accurately weighed bismuth to place in a 100ml volumetric flask; adding 6.4ml nitric acid to dissolve the bismuth; diluting with water until the scale is reached; thus, a standard bismuth stock solution is prepared; accurately weighing 1ml of standard bismuth stock solution to place in a 100ml volumetric flask; diluting with a hydrochloric acid solution of 0.8mol/L until the scale is reached; thus, a solution containing about 50µg bismuth in each 1ml is prepared as the standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 110mg content in a colloidal bismuth pectin capsule to place in a 100ml volumetric flask; adding 50ml of water; shaking or impacting with an ultrasonic treatment to make a solution dissolve evenly; diluting with water until a scale is reached; thus, a dispersion containing about 100µg bismuth in each 1ml is prepared as a test stock solution; accurately weighing 5ml of the test stock solution to place in a 15ml centrifugal tube; accurately adding 5ml of 1.6mol/L hydrochloric acid solution; shaking for 5 minutes; centrifuging for 10 minutes at 7000r/min; accurately measuring 5ml supernatant to place in a 25ml volumetric flask; diluting with chromogen solution until a scale is reached; thus, the test solution is prepared.
   4) Preparation of a blank solution: accurately measuring 5ml of water to place in a 15ml centrifugal tube; accurately adding 5ml hydrochloric acid solution of 1.6mol/L; shaking for 5 minutes; accurately measuring 5ml solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying a ultraviolet-visible spectrophotometry, comprising steps of using 1cm quartz cuvette; measuring an absorbance at 463nm wavelength; calculating a colloidal bismuth pectin content with an external standard method; a result is 98.4% of a labeled amount(take bismuth for calculation).
Embodiment 4: colloidal bismuth pectin capsule (specification: 100mg, take the bismuth for calculation).
   1) Preparation of a chromogen solution: taking 2g citric acid and 20g potassium iodide to place in a volumetric flask of 200ml; adding 100ml of water; shaking to dissolve; adding water to dilute; dripping water to meet a scale; thus, the chromogen solution containing 1% of a citric acid, 10% of a potassium iodide is prepared.
   2) Preparation of a reference solution of bismuth: taking 275mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 1.5ml of standard bismuth stock solution to place in a 100ml volumetric flask; diluting with a sulfuric acid solution of 0.5mol/L until a scale is reached; thus, a solution containing about 41.25µg bismuth in each 1ml is prepared as a standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 63.7mg content in a colloidal bismuth pectin capsule to place in a 100ml volumetric flask; adding 50ml of water; shaking or impacting with an ultrasonic treatment to make the solution dissolve evenly; diluting with water until a scale is reached; thus, a dispersion containing about 82.5µg bismuth in each 1ml is prepared as a test stock solution; accurately weighing 5ml of test stock solution to place in a 15ml centrifugal tube; accurately adding 5ml of 1 mol/L sulfuric acid solution; shaking for 5 minutes; centrifuging for 10 minutes at 10000r/min; accurately measuring 5ml of supernatant to place in a 25ml volumetric flask; diluting with chromogen solution until a scale is reached; thus, the test solution is prepared.
   4) Preparation of a blank solution: accurately measuring 5ml of water to place in a 15ml centrifugal tube; accurately adding 5ml of 1mol/L sulfuric acid solution; shaking for 5 minutes; accurately measuring 5ml of a solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying an ultraviolet-visible spectrophotometry, comprising steps of using 1cm quartz cuvette; measuring an absorbance at 433nm and 463nm wavelength; calculating a colloidal bismuth pectin content with an external standard method according to an absorbance difference at the two wavelength; a result is 99.6% of a labeled amount (take bismuth for calculation).
Embodiment 5: colloidal bismuth pectin dispersible tablet (specification: 50mg, take the bismuth for calculation).
   1) Preparation of a chromogen solution: taking 20g citric acid and 50g potassium iodide to place in a volumetric flask of 200ml; adding 100ml of water; shaking to dissolve; adding 25ml of 5 mol/L nitric acid solution; dripping water to meet a scale; thus, the chromogen solution containing 10% of a citric acid, 25% of a potassium iodide is prepared.
   2) Preparation of a reference solution of bismuth: taking 275mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 0.4ml of standard bismuth stock solution to place in a 100ml volumetric flask; diluting with a nitric acid solution of 0.9mol/L until a scale is reached; thus, a solution containing about 11µg bismuth in each 1ml is prepared as a standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: taking 20 colloidal bismuth pectin dispersible tablets to finely grind; accurately weighing 33.8mg of the powder to place in a 100ml volumetric flask; adding 50ml of water; shaking or impacting with an ultrasonic treatment to make a solution dissolve evenly; diluting with water until a scale is reached; thus, a dispersion containing about 22µg bismuth in each 1ml is prepared as a test stock solution; accurately weighing 5ml test stock solution to place in a 15ml centrifugal tube; accurately adding 5ml of 1.8 mol/L nitric acid solution; shaking for 5 minutes; centrifuging for 10 minutes at 8000r/min; accurately measuring 5ml of supernatant to place in a 25ml volumetric flask; diluting with the chromogen solution until a scale is reached; thus, the test solution is prepared.
   4) Preparation of a blank solution: accurately measuring 5ml of water to place in a 15ml centrifugal tube; accurately adding 5ml of 1.8mol/L nitric acid solution; shaking for 5 minutes; accurately measuring 5ml solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying a ultraviolet-visible spectrophotometry, comprising steps of using 1cm quartz cuvette; measuring an absorbance at 399nm wavelength; calculating a colloidal bismuth pectin content with an external standard method; a result is 99.0% of a labeled amount(take bismuth for calculation).
Embodiment 6: colloidal bismuth pectin granules (specification: 150mg, take the bismuth for calculation).
   1) Preparation of a chromogen solution: taking 8g ascorbic acid and 30g potassium iodide to place in a volumetric flask of 200ml; adding 100ml of water; shaking to dissolve; adding 25ml of lmol/L nitric acid solution; adding water to dilute; dripping water to meet a scale; thus, the chromogen solution containing 4% of an ascorbic acid, 15% of a potassium iodide is prepared.
   2) Preparation of a reference solution of bismuth: taking 50mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 1ml standard bismuth stock solution to place in a 100ml volumetric flask; diluting with a nitric acid solution of 1.1mol/L until a scale is reached; thus, a solution containing about 5µg bismuth in each 1ml is prepared as the standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: taking the colloidal bismuth pectin granules to finely grind; accurately weighing 19mg of the powder to place in a 100ml volumetric flask; adding 50ml of water; shaking or impacting with an ultrasonic treatment to make the solution dissolve evenly; diluting with water until a scale is reached; thus, a dispersion containing about 10µg bismuth in each 1ml is prepared as a test stock solution; accurately weighing 5ml test stock solution to place in a 15ml centrifugal tube; accurately adding 5ml of 2.2 mol/L nitric acid solution; shaking for 5 minutes; centrifuging for 15 minutes at 9000r/min; accurately measuring 5ml supernatant to place in a 25ml volumetric flask; diluting with chromogen solution until a scale is reached; thus, the test solution is prepared.
   4) Preparation of a blank solution: accurately measuring 5ml of water to place in a 15ml centrifugal tube; accurately adding 5ml of 2.2mol/L nitric acid solution; shaking for 5 minutes; accurately measuring 5ml of a solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying an ultraviolet-visible spectrophotometry, comprising steps of using 1cm quartz cuvette; measuring an absorbance at 463nm wavelength; calculating a colloidal bismuth pectin content with an external standard method; the result is 99.0% of a labeled amount(take bismuth for calculation).
Embodiment 7: compound colloidal bismuth pectin capsule (comprising colloidal bismuth pectin, metronidazole and tetracycline hydrochloride; every capsule containing 35mg colloidal bismuth pectin which is calculated with bismuth).
   1) Preparation of a chromogen solution: taking 15g ascorbic acid and 40g potassium iodide to place in a volumetric flask of 200ml; adding 100ml of water; shaking to dissolve; adding 25ml of 1mol/L nitric acid solution; adding water to dilute; dripping water to meet a scale; thus, the chromogen solution containing 7.5% of an ascorbic acid, 20% of a potassium iodide is prepared.
   2) Preparation of a reference solution of bismuth: taking 275mg accurately weighed bismuth to place in a 100ml volumetric flask; adding 6.4ml nitric acid to dissolve the bismuth; diluting with water until a scale is reached; thus, the standard bismuth stock solution is prepared; accurately weighing 1ml of standard bismuth stock solution to place in a 100ml volumetric flask; diluting with a nitric acid solution of 0.8mol/L until a scale is reached; thus, a solution containing about 27.5µg bismuth in each 1ml is prepared as the standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 184mg content of the compound colloidal bismuth pectin dispersible capsules to place in a 100ml volumetric flask; adding 50ml of water; shaking or impacting with an ultrasonic treatment to make the solution dissolve evenly; diluting with water until a scale is reached; thus, a dispersion containing about 55µg bismuth in each 1ml is prepared as the test stock solution; accurately weighing 5ml of test stock solution to place in a 15ml centrifugal tube; accurately adding 5ml of 1.6 mol/L nitric acid solution; shaking for 5 minutes; centrifuging for 5 minutes at 10000r/min; accurately measuring 5ml of supernatant to place in a 25ml volumetric flask; diluting with the chromogen solution until a scale is reached; thus, the test solution is prepared.
   4) Preparation of a blank solution: accurately measuring 5ml of water to place in a 15ml centrifugal tube; accurately adding 5ml of 1.6mol/L nitric acid solution; shaking for 5 minutes; accurately measuring 5ml solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying a ultraviolet-visible spectrophotometry, comprising steps of using 1cm quartz cuvette; measuring an absorbance at 399nm and 463nm wavelength; calculating the colloidal bismuth pectin content with an external standard method according to the absorbance difference at the two wavelength; a result is 101.3% of a labeled amount(take bismuth for calculation).
Embodiment 8: colloidal bismuth pectin powder (specification: 150mg, take the bismuth for calculation).
   1) Preparation of a chromogen solution: taking 1g ascorbic acid and 5g potassium iodide to place in a volumetric flask of 200ml; adding 100ml of water; shaking to dissolve; adding 25ml of 1mol/L nitric acid solution; adding water to dilute; dripping water to meet a scale; thus, the chromogen solution containing 0.5% of ascorbic acid, 2.5% of potassium iodide is prepared.
   2) Preparation of a reference solution of bismuth: taking 10mg accurately weighed bismuth to place in a 100ml volumetric flask; adding 6.4ml nitric acid to dissolve the bismuth; diluting with water until a scale is reached; thus, a standard bismuth stock solution is prepared; accurately weighing 1ml of standard bismuth stock solution to place in a 100ml volumetric flask; diluting with a hydrochloric acid solution of 1mol/L until a scale is reached; thus, a solution containing about 1µg bismuth in each 1ml is prepared as a standard bismuth solution; accurately weighing 5ml standard bismuth solution to place in a 25ml volumetric flask; diluting with the chromogen solution until a scale is reached; thus, the reference solution of bismuth is prepared.
   3) Preparation of a test solution: accurately weighing 10mg of the colloidal bismuth pectin powder to place in a 500ml volumetric flask; adding 50ml of water; shaking or impacting with an ultrasonic treatment to make the solution dissolve evenly; diluting with water until a scale is reached; thus, a dispersion containing about 2µg bismuth in each 1ml is prepared as the test stock solution; accurately weighing 5ml test stock solution to place in a 15ml centrifugal tube; accurately adding 5ml of 1.9 mol/L hydrochloric acid solution; shaking for 5 minutes; centrifuging for 10 minutes at 8000r/min; accurately measuring 5ml supernatant to place in a 25ml volumetric flask; diluting with chromogen solution until a scale is reached; thus, the test solution is prepared.
   4) Preparation of a blank solution: accurately measuring 5ml of water to place in a 15ml centrifugal tube; accurately adding 5ml 1.9mol/L hydrochloric acid solution; shaking for 5 minutes; accurately measuring 5ml of the solution to place in a 25ml volumetric flask; diluting with the chromogen solution until a scale is reached; thus the blank solution is prepared.
   5) Measuring: taking the reference solution and the test solution of the bismuth; taking the blank solution as a reference; applying an ultraviolet-visible spectrophotometry, comprising steps of using 1cm quartz cuvette; measuring an absorbance at 463nm wavelength; calculating the colloidal bismuth pectin content with an external standard method; a result is 99.0% of a labeled amount(take bismuth Bi for calculation).
Embodiment 9: test for accuracy.
   Taking the bismuth reference solution; testing the absorbance at 463nm wavelength; repeating the test for 6 times, wherein the results are 0.6494, 0.6494, 0.6495, 0.6494, 0.6494, 0.6495 respectively; the RSD (relative standard deviation) is 0.1%; the accuracy of the device is good.
Embodiment 10: linear range
   Preparing a series of standard bismuth solution concentrations of which are 87.5µg/ml, 55µg/ml, 27.5µg/ml, 11µg/ml, 8.8µg/ml, 5.5µg/ml, 2.2µg/ml, 1.1µg/ml, 0.55µg/ml respectively; accurately weighing 5ml of each of the series of standard bismuth solution to place in a 25ml volumetric flask; diluting with the chromogen solution until a scale is reached; measuring an absorbance at the 463nm wavelength; using the least square to linearly regress an absorbance and the concentration of the standard bismuth solution; a regression formula is A=0.0114C+0.0032; a relative coefficient is R²=0.9999; the standard bismuth solution shows good linear relationship within a concentration range of 0.55-87.5µg/mL.
Embodiment 11: limit of detection and limit of quantification.
   Preparing 20 blank solutions and measuring an absorbance at 463nm wavelength, wherein absorbance are 0.0434, 0.0449, 0.0441, 0.0451, 0.0436, 0.0424, 0.0438, 0.0434, 0.0451, 0.0445, 0.0444, 0.0445, 0.0443, 0.0455, 0.0441, 0.0446, 0.0444, 0.0443, 0.0441 and 0.0445 respectively; a calculation standard deviation SD equals 0.00070; according to the regulation of International Union of Pure and Applied Chemistry (IUPAC) on the limit of detection, a calculated limit of detection =3×0.00070=0.0021 which is equivalent to a standard bismuth solution of 0.15µg/ml; a limit of quantification=10×0.00070=0.0070 which is equivalent to a standard bismuth solution of 0.5µg/ml.
Embodiment 12: recovery rate.
   Taking proper amount of the content in a colloidal bismuth pectin capsule (equivalent to 2.75mg bismuth) to place in 100ml volumetric flasks; adding 0.8ml, 1ml, 1.2ml of the standard bismuth stock solution (2.75mg/ml) respectively; adding 50ml water and impacting with ultrasonic treatment to make the solution evenly dispersed; diluting with water until a scale is reached; thus, the stock solution is prepared.
   Taking 5ml stock solution to place in a 10ml centrifugal tube; accurately adding 5ml of 2mol/L nitric acid solution; shaking and centrifuging; accurately measuring 5ml of supernatant to place in a 25ml volumetric flask; diluting with the chromogen solution until a scale is reached; repeatedly preparing 3 solutions for each stock solutions and measuring an absorbance at 463nm wavelength; a calculated recovery rates are 99.28%, 100.69%, 99.77%, 97.93%, 101.57%, 99.16%, 99.40%, 100.86% and 99.64% respectively; an average recovery rate is 99.81% and RSD=1.09%, which shows the method has good recovery rate and accuracy.
Embodiment 13: repeatability.
   Preparing 6 bismuth reference solutions and test solutions respectively and measuring an absorbance; absorbance of reference solutions are 0.6537, 0.6479, 0.6487, 0.6509, 0.6539 and 0.6531 respectively, RSD=0.4%; contents of the test article are 98.40%, 98.74%, 98.66%, 98.35%, 98.55% and 98.62% of a labeled amount respectively, RSD=0.2%; the method has good repeatability.
Embodiment 14: the stability of the solution.
   Taking the test solution and measuring an absorbance in 0, 2, 4, 6, 8 hours, wherein absorbance are 0.6528, 0.6558, 0.6489, 0.6514, 0.6504 and 0.6538 respectively, RSD=0.4%; the test solution has good stability within 8 hours.
   Accurately weighing the proper amount of the content in a colloidal bismuth pectin capsule (about 5.5mg bismuth) to place in a 100ml volumetric flask; adding 50ml of water; impacting with an ultrasonic treatment to disperse the solution evenly; diluting with water until a scale is reached; taking 5ml of the colloidal solution in 0, 2, 4, 6, 8 hours to place in a 10ml centrifugal tube; accurately adding 5ml of 2mol/L nitric acid solution; shaking and centrifuging for 10 minutes at 8000r/min; accurately taking 5ml supernatant to place in a 25ml volumetric flask; diluting with a chromogen solution until a scale is reached; measuring absorbance and calculating bismuth contents which are 98.79%, 98.27%, 98.55%, 98.19%, 98.66% and 98.82% of a labeled amount respectively, RSD=0.3%; a stability of a colloidal solution is good within 8 hours and the colloidal solution is able to keep in a homogeneous state.
Embodiment 15: contrast test for ultraviolet-visible spectrophotometry and complexometric titration.

### 1. Complexometric titration

Accurately weighing 0.5g colloidal bismuth pectin, adding 5ml nitric acid solution (1→2); dissolving a solution by heating; adding 150ml of water and two drops of xylenol orange indicator; titrating the solution with EDTA-2Na (ethylenediaminetetraacetic acid disodium salt dehydrate) volumetric solution of 0.05mol/L until the solution appears yellow; each 1ml EDTA-2Na volumetric solution is equivalent to 10.45mg bismuth(Bi); testing in parallel for 6 times, wherein results are 15.26%, 14.87%, 15.14%, 14.72%, 14.69% and 14.93% respectively, averaged 14.94%, RSD%=1.52%.

Accurately weighing 0.25g colloidal bismuth pectin; adding 5ml nitric acid solution (1→2); dissolving a solution by heating; adding 1.2ml, 1.5ml, 1.8ml of standard bismuth stock solutions (27.5mg/ml); adding 150ml of water and two drops of xylenol orange indicator; titrating the solution with EDTA-2Na volumetric solution of 0.05mol/L until the solution appears yellow; each 1ml EDTA-2Na volumetric solution is equivalent to 10.45mg bismuth Bi; repeatedly preparing 3 of each of the solution for test; calculated recovery rates are 102.95%, 98.13%, 101.64%, 103.76%, 98.07%, 99.12%, 101.69%, 103.26% and 102.31% respectively, an averaged recovery rate is 101.21%, RSD=2.18%.

### 2. Ultraviolet-visible spectrophotometry.

Accurately weighing 37mg colloidal bismuth pectin; testing in parallel according to the embodiment 1 for 6 times, wherein the results are 14.86%, 14.95%, 14.90%, 14.88%, 14.99% and 14.83% respectively, averaged 14.90%, RSD%=0.40%.

Taking 37mg colloidal bismuth pectin to place in 100ml volumetric flasks; adding 1.6ml, 2.0ml and 2.4ml standard bismuth stock solution (2.75mg/ml) respectively; adding 50ml of water and impacting with the ultrasonic treatment to disperse the solution evenly; diluting with water until a scale is reached; thus, the stock solution is prepared; taking 5ml of the stock solution to place in a 10ml centrifugal tube; accurately adding 5ml nitric acid solution of 2mol/L; shaking and centrifuging; accurately measuring 5ml supernatant to place in a 25ml volumetric flask; diluting with the chromogen solution until a scale is reached; repeatedly prepare 3 parts of each stock solution; measuring an absorbance at 463nm wavelength; calculated recovery rates are 98.89%, 101.38%, 99.86%, 101.71%, 100.87%, 99.09%, 101.25%, 100.91% and 100.71% respectively; an average recovery rate is 100.52%, RSD=1.00%.

The present invention solves the difficulty in complexometric titration of end point detection. The recovery rate, repeatability and other features of the measurement are superior to the complexometric titration.

## Claims

1. A method for measuring a bismuth content in a colloidal bismuth pectin or a colloidal bismuth pectin containing preparation, comprising the steps of: dispersing the colloidal bismuth pectin or the colloidal bismuth pectin containing preparation into water; adding a protonic acid dissociation agent into the dispersion until a hydrogen ion concentration reaches 0.8mol/L-1.2mol/L; centrifuging the dispersion after complete dissociation; seperating a supernatant; coloring the supernatant by adding a chromogen solution of citric acid or ascorbic acid and potassium iodide to form a test solution; testing an absorbance of the test solution at a wavelength of 380nm-470nm; comparing the absorbance of the test solution with an absorbance of a reference solution of a known bismuth concentration under the same conditions; calculating the bismuth content in the colloidal bismuth pectin or the colloidal bismuth pectin containing preparation.

2. The method as recited in claim 1, wherein the protonic acid dissociation agent is nitric acid, hydrochloric acid or sulfuric acid.

3. The method as recited in claim 2, wherein the protonic acid dissociation agent is nitric acid.

4. The method as recited in claim 1, wherein the chromogen solution is a water solution or a 0.2mol/L-2mol/L nitric acid solution of citric acid or ascorbic acid and potassium iodide.

5. The method as recited in claim 4, wherein the chromogen solution comprises citric acid or ascorbic acid of 0.5wt%-10wt%, and potassium iodide of 2.5wt%-25wt%.

6. The method as recited in claim 4, wherein the chromogen solution comprises citric acid or ascorbic acid of 2.5 wt%, and potassium iodide of 12.5wt%.

7. The method as recited in claim 1, wherein the reference solution of bismuth is prepared by dissolving bismuth with nitric acid before being diluted with water and adding the chromogen solution.

8. The method as recited in claim 1, wherein in each 1ml test solution or ml reference solution of the bismuth, a bismuth content is 0.1 µg-50µg.

9. The method as recited in claim 1, wherein in each 1ml test solution or 1ml reference solution of the bismuth, the bismuth content is 2µg-20µg.

10. The method as recited in claim 1, wherein in each 1ml test solution or 1ml reference solution of the bismuth, the bismuth content is 5µg-12µg.

11. The method as recited in claim 1, wherein a single-wavelength method is adopted for the absorbance measurement; wherein the detection wavelength is chosen to be any one wavelength from 399nm, 433nm and 463nm.

12. The method as recited in claim 11, wherein the detection wavelength is 463nm.

13. The method as recited in claim 1, wherein a double-wavelength method is adopted for the absorbance measurement; wherein the detection wavelengths are chosen to be any two wavelengths from 399nm, 433nm and 463nm.

14. The method as recited in claim 13, wherein the detection wavelengths are 433nm and 463nm.

15. The method as recited in claim 1, wherein the colloidal bismuth pectin containing preparation is a single preparation or a compound preparation selected from tablets, dispersible tablets, eneric-coated tablets, colon-enteric-coated tablets, capsules, soft capsules, eneric-coated capsules, colon-enteric-coated capsules, granules, dripping pills, microcapsules and dry suspensions.

## Patentansprüche

1. Verfahren zur Messung eines Bismutgehalts in kolloidalem Bismutpektin oder in einem Präparat, das kolloidales Bismutpektin enthält, folgende Schritte aufweisend:
Dispergieren des kolloidalen Bismutpektins oder des Präparats, das kolloidales Bismutpektin enthält, in Wasser; Zugeben eines Protonendonator-Dissoziationsmittels in die Dispersion bis die Wasserstoffionen-Konzentration 0,8 Mol/l bis 1,2 Mol/l erreicht; Zentrifugieren der Dispersion nach vollständiger Dissoziation; Abtrennen eines Überstands; Färben des Überstands durch Zugeben einer Chromogen-Lösung von Zitronensäure oder Ascorbinsäure und Kaliumiodid, um eine Testlösung herzustellen; Testen der Extinktion der Testlösung bei einer Wellenlänge von 380 nm bis 470 nm; Vergleichen der Extinktion der Testlösung mit der Extinktion einer Referenzlösung mit einer bekannten Bismut-Konzentration unter denselben Bedingungen; Berechnen des Bismutgehalts in dem kolloidalen Bismutpektin oder in dem Präparat, das kolloidales Bismutpektin enthält.

2. Verfahren wie in Anspruch 1 angegeben, wobei das Protonendonator-Dissoziationsmittel Salpetersäure, Salzsäure oder Schwefelsäure ist.

3. Verfahren wie in Anspruch 2 angegeben, wobei das Protonendonator-Dissoziationsmittel Salpetersäure ist.

4. Verfahren wie in Anspruch 1 angegeben, wobei die Chromogen-Lösung eine Wasserlösung oder eine 0,2 Mol/l bis 2 Mol/l Salpetersäure-Lösung von Zitronensäure oder Ascorbinsäure und Kaliumiodid ist.

5. Verfahren wie in Anspruch 4 angegeben, wobei die Chromogen-Lösung 0,5 Gew.-% bis 10 Gew.-% Zitronensäure oder Ascorbinsäure und 2,5 Gew.-% bis 25 Gew.-% Kaliumiodid aufweist.

6. Verfahren wie in Anspruch 4 angegeben, wobei die Chromogen-Lösung 2,5 Gew.-% Zitronensäure oder Ascorbinsäure und 12,5 Gew.-% Kaliumiodid aufweist.

7. Verfahren wie in Anspruch 1 angegeben, wobei die Referenzlösung des Bismuts hergestellt wird durch Auflösen des Bismuts mit Salpetersäure vor dem Verdünnen mit Wasser, und Zugeben der Chromogen-Lösung.

8. Verfahren wie in Anspruch 1 angegeben, wobei der Bismutgehalt in jeweils 1 ml Testlösung oder 1 ml Referenzlösung des Bismuts 0,1 µg bis 50 µg ist.

9. Verfahren wie in Anspruch 1 angegeben, wobei der Bismutgehalt in jeweils 1 ml Testlösung oder 1 ml Referenzlösung des Bismuts 2 µg bis 20 µg ist.

10. Verfahren wie in Anspruch 1 angegeben, wobei der Bismutgehalt in jeweils 1 ml Testlösung oder 1 ml Referenzlösung des Bismuts 5 µg bis 12 µg ist.

11. Verfahren wie in Anspruch 1 angegeben, wobei zur Extinktionsmessung ein Verfahren mit einer einzigen Wellenlänge verwendet wird, wobei als Nachweiswellenlänge irgendeine der Wellenlängen 399 nm, 433 nm und 463 nm gewählt wird.

12. Verfahren wie in Anspruch 11 angegeben, wobei die Nachweiswellenlänge 463 nm ist.

13. Verfahren wie in Anspruch 1 angegeben, wobei zur Extinktionsmessung ein Verfahren mit zwei Wellenlängen verwendet wird; wobei als Nachweiswellenlängen beliebige zwei Wellenlängen der Wellenlängen 399 nm, 433 nm und 463 nm gewählt werden.

14. Verfahren wie in Anspruch 13 angegeben, wobei die Nachweiswellenlängen 433 nm und 463 nm sind.

15. Verfahren wie in Anspruch 1 angegeben, wobei das Präparat, das das kolloidale Bismutpektin enthält, ein Einzelpräparat oder ein Kombinationspräparat ist, das ausgewählt ist aus Tabletten, dispergierbaren Tabletten, magensaftresistent-beschichteten Tabletten, colon-magensaftresistent-beschichteten Tabletten, Kapseln, Weichkapseln, magensaftresistent-beschichteten Kapseln, colon-magensaftresistent-beschichteten Kapseln, Granulat, Tropfpillen, Mikrokapseln und Trockensäften.

## Revendications

1. Procédé destiné à mesurer une teneur en bismuth dans une pectine de bismuth colloïdal ou une préparation contenant une pectine de bismuth colloïdal, comprenant les étapes consistant à :
disperser la pectine de bismuth colloïdal ou la préparation contenant une pectine de bismuth colloïdal dans de l'eau ;
ajouter un agent de dissociation d'acide protonique dans la dispersion jusqu'à ce qu'une concentration en ions hydrogène atteigne 0,8 mol/L-1,2 mol/L ;
centrifuger la dispersion après dissociation complète ;
séparer un surnageant ;
colorer le surnageant en ajoutant une solution chromogène d'acide citrique ou d'acide ascorbique et d'iodure de potassium afin de former une solution d'essai ;
tester une absorbance de la solution d'essai à une longueur d'onde de 380 nm-470 nm ;
comparer l'absorbance de la solution d'essai à une absorbance d'une solution de référence d'une concentration en bismuth connue sous les mêmes conditions, et
calculer la teneur en bismuth dans la pectine de bismuth colloïdal ou la préparation contenant une pectine de bismuth colloïdal.

2. Procédé selon la revendication 1, dans lequel l'agent de dissociation d'acide protonique est de l'acide nitrique, de l'acide chlorhydrique, ou de l'acide sulfurique.

3. Procédé selon la revendication 2, dans lequel l'agent de dissociation d'acide protonique est de l'acide nitrique.

4. Procédé selon la revendication 1, dans lequel la solution chromogène est une solution aqueuse ou une solution d'acide nitrique à 0,2 mol/L-2 mol/L d'acide citrique ou d'acide ascorbique et d'iodure de potassium.

5. Procédé selon la revendication 4, dans lequel la solution chromogène comprend de l'acide nitrique ou de l'acide ascorbique à 0,5 % en poids-10 % en poids et de l'iodure de potassium à 2,5 % en poids-25 % en poids.

6. Procédé selon la revendication 4, dans lequel la solution chromogène comprend de l'acide nitrique ou de l'acide ascorbique à 2,5 % en poids et de l'iodure de potassium à 12,5 % en poids.

7. Procédé selon la revendication 1, dans lequel la solution de référence de bismuth est préparée en dissolvant du bismuth avec de l'acide nitrique avant d'être diluée avec de l'eau et d'ajouter la solution chromogène.

8. Procédé selon la revendication 1, dans lequel dans chaque 1 ml de solution d'essai ou 1 ml de solution de référence du bismuth, une teneur en bismuth s'élève à 0,1 µg-50 µg.

9. Procédé selon la revendication 1, dans lequel dans chaque 1 ml de solution d'essai ou 1 ml de solution de référence du bismuth, la teneur en bismuth s'élève à 2 µg-20 µg.

10. Procédé selon la revendication 1, dans lequel dans chaque 1 ml de solution d'essai ou 1 ml de solution de référence du bismuth, la teneur en bismuth s'élève à 5 µg-12 µg.

11. Procédé selon la revendication 1, dans lequel un procédé à une seule longueur d'onde est adopté pour la mesure d'absorbance, et
dans lequel la longueur d'onde de détection est choisie pour être une longueur d'onde quelconque parmi 399 nm, 433 nm et 463 nm.

12. Procédé selon la revendication 11, dans lequel la longueur d'onde de détection est 463 nm.

13. Procédé selon la revendication 1, dans lequel un procédé à double longueur d'onde est adopté pour la mesure d'absorbance, et
dans lequel les longueurs d'onde de détection sont choisies pour être deux longueurs d'onde quelconques parmi 399 nm, 433 nm et 463 nm.

14. Procédé selon la revendication 13, dans lequel les longueurs d'onde de détection sont 433 nm et 463 nm.

15. Procédé selon la revendication 1, dans lequel la préparation contenant une pectine de bismuth colloïdal est une préparation unique ou une préparation composée sélectionnée parmi des comprimés, des comprimés dispersibles, des comprimés gastro-résistants, des comprimés entérosolubles, des capsules, des capsules molles, des capsules gastrorésistantes, des capsules entérosolubles, des granulés, des pilules encapsulées, des microcapsules, et des suspensions sèches.
